(19) Europäisches Patentamt / European Patent Office / Office européen des brevets

(11) **EP 1 842 559 B1**

(12) **EUROPEAN PATENT SPECIFICATION**

(45) Date of publication and mention of the grant of the patent:
**25.08.2010 Bulletin 2010/34**

(51) Int Cl.:
*A61K 41/00* (2006.01)    *A61K 9/127* (2006.01)
*A61K 31/685* (2006.01)    *A61P 35/00* (2006.01)

(21) Application number: **05781870.0**

(22) Date of filing: **02.09.2005**

(86) International application number:
**PCT/CN2005/001392**

(87) International publication number:
**WO 2006/072201 (13.07.2006 Gazette 2006/28)**

(54) **PARTICLE ADJUVANT FOR HIFU THERAPY AND THE USE THEREOF**

TEILCHENFÖRMIGES ADJUVANS FÜR HIFU-THERAPIE UND SEINE VERWENDUNG

ADJUVANT SOUS FORME DE PARTICULES POUR UN TRAITEMENT HIFU ET SON UTILISATION

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.01.2005 CN 200510000348**

(43) Date of publication of application:
**10.10.2007 Bulletin 2007/41**

(73) Proprietor: **Chongqing Haifu (Hifu) Technology Co., Ltd.**
**Chongqing 401121 (CN)**

(72) Inventors:
- **WANG, Zhibiao**
  **Yubei District**
  **401121 Chongqing (CN)**
- **LI, Faqi**
  **Jiulongpo District,**
  **400041 Chongqing (CN)**
- **XIAO, Yanbing**
  **Jiulongpo District,**
  **400041 Chongqing (CN)**
- **XIAO, Ziwen**
  **Jiulongpo District,**
  **400041 Chongqing (CN)**
- **LIU, Liping**
  **Jiulongpo District,**
  **400041 Chongqing (CN)**
- **WANG, Zhilong**
  **Jiulongpo District,**
  **400041 Chongqing (CN)**

(74) Representative: **Kampfenkel, Klaus et al**
**Blumbach - Zinngrebe**
**Patentanwälte**
**Alexandrastrasse 5**
**65187 Wiesbaden (DE)**

(56) References cited:
**CN-A- 1 073 880**    **CN-A- 1 380 106**
**CN-A- 1 404 878**    **JP-A- 58 032 829**
**US-B1- 6 551 576**

- **DATABASE BIOSIS BIOSCIENCES INFORMATION SERVICE, PHILADELPHIA, PA, US; Nippon Acta Radiologica 1998, TAKEO I. ET AL.: "Usefulness of CO2 US angiography in treating hepatocellular carcinoma" XP007903959**
- **SHU-QUN CHENG ET AL: "Iodized oil enhances the thermal effect of high-intensity focused ultrasound on ablating experimental liver cancer" JOURNAL OF CANCER RESEARCH AND CLINICAL ONCOLOGY, SPRINGER-VERLAG, BE, vol. 123, 1997, pages 639-644, XP007903958 ISSN: 1432-1335**
- **GENNARO A. R. ET AL.: ""Remington: the Science and Practice of Pharmacy 20th edition"" 2000, LINPINNCOTT WILLIAMS AND WILKINS , BALTIMORE USA , XP007903936 * page 847; tables 44-4 ***
- **HOPMAN W.P.M. ET AL.: "Role of Cholecystokinin and the Cholinergic System in Intestinal Stimulation of Gallbladder Contraction in Man" HEPATOLOGY, vol. 11, no. 2, 1990, pages 261-265, XP007903952**

• HARVEY C J ET AL: "ADVANCES IN ULTRASOUND" CLINICAL RADIOLOGY, LIVINGSTONE, HARLOW,, GB, vol. 57, no. 3, March 2002 (2002-03), pages 157-177, XP009071168 ISSN: 0009-9260

• JI XIAOJUAN ET AL.: 'the use of ultrasound contrast-agent in HIFU against tumors' CHINA J ULTRASONOGR vol. 12, no. 12, December 2003, pages 748 - 750, XP008091713

**Description**

**FIELD OF THE PRESENT INVENTION**

The present invention is related to the fields of medicine and medical treatment, specifically to the field of ultrasound treatment, and more particularly to a particle enhancement agent for HIFU treatment, which can increase acoustic energy deposition at the target location during HIFU treatment, and use thereof.

**BACKGROUND OF THE PRESENT INVENTION**

High-intensity focused ultrasound (HIFU) as a new technique to treat tumors and other diseases has already been recognized in clinical applications. HIFU employs focused ultrasound, which provides continuous, high-intensity ultrasound energy at the focus, resulting in instantaneous thermal effects (65-100°C), cavitation effects, mechanical effects and sonochemical effects, to selectively cause coagulative necrosis at the focus, and prevent tumors from proliferation, invasion and metastasis.

Cheng Shu-Qun et al. disclosed the use of iodized oil in HIFU treatment of liver cancer (Journal of Cancer Research and Clinical Oncology, Springer-Verlag, BE, vol. 123, 1997, pages 639-644, XP007903958, ISSN: 1432-1335).

It was demonstrated that the acoustic energy was attenuated exponentially as the transmission distance increased during ultrasound transmission within the body (Baoqin Liu et al., Chinese Journal of Ultrasound in Medicine, 2002, 18(8):565-568.

In addition, the energy during ultrasound transmission in soft tissues was attenuated due to tissue absorption, scattering, refraction, diffraction and the like, among which the tissue absorption and scattering are mainly responsible for the energy loss (Ruo Feng and Zhibiao Wang as editors in chief, *Practical Ultrasound Therapeutics,* Science and Technology Reference Publisher of China, Beijing, 2002.14). Therefore, when the HIFU treatment is used to treat deep-seated and large-sized tumors, the acoustic energy transmitted to the target would be relatively low. Thus, therapeutic efficiency would decrease and treatment time would be prolonged due to the acoustic energy attenuation.

Of course, although the transmitting power of the therapeutic transducer might be increased in order to improve the therapeutic efficiency, the normal tissue along the pathway of the ultrasound transmission is more likely to be burned in a high-intensity ultrasound environment.

In addition, at present, when the HIFU technique is clinically applied to a hepatic tumor that is blocked by the ribs in the pathway of the ultrasound transmission, the ribs are usually removed in order to increase the energy deposition at the target location, shorten the treatment time and improve therapeutic effects. Thus the noninvasiveness of HIFU treatment cannot be ensured, which is undesirable for the patients and doctors.

The above problems have disadvantageously limited the use of the HIFU treatment as a technique for clinical practice. Therefore, the technical problems with respect to increasing the energy deposition at target location, effectively treating the deep-seated tumors without damaging the surrounding normal tissue in the acoustic pathway, and treating a hepatic tumor that is blocked by the ribs without removal of the ribs, need to be solved urgently.

**SUMMARY OF THE INVENTION**

One objective of the present invention is to provide a particle enhancement agent for HIFU treatment, which can enhance the acoustic energy deposition at target tissue during HIFU treatment.

Another objective of the present invention is to provide a method for enhancing acoustic energy deposition at the target location during HIFU treatment using the particle enhancement agent for HIFU treatment of the present invention.

A further objective of the present invention is to provide use of a particle enhancement agent for HIFU treatment to enhance the effectiveness of HIFU treatment.

In order to achieve the above objectives, in one embodiment, the present invention provides an enhancement agent for use in HIFU treatment according to the claims. The enhancement agent of the present invention is a substance that can enhance the acoustic energy absorption at target location to be treated with HIFU after its administration to a biological body, i.e. a substance that can be used to reduce the acoustic energy needed to cause lesions of a target tissue (tumor and non-tumor tissue) per unit volume of the tissue during HIFU treatment.

As used herein, the term "lesion" refers to the substantial change in the physiological state of a tumor or normal tissue, generally refers to the coagulative necrosis of a tumor or normal tissue. Energy efficiency factor (EEF) can be used to quantify the acoustic energy needed to cause lesions of a target tissue per unit volume of the tissue. EEF is presented by the expression $EEF = \eta Pt/V$ (unit: J/mm$^3$), and refers to the acoustic energy needed to cause lesions of a tumor or normal tissue per unit volume of the tissue, wherein, η refers to the focusing coefficient of a HIFU transducer, which reflects the ultrasound energy focusing capacity of the transducer, here η=0.7; P refers to the total acoustic power of a HIFU source (unit: W), t refers to the total time of HIFU treatment (unit: s); and V refers to the volume of HIFU-induced lesions (unit: $mm^3$). A substance that greatly decreases the EEF of the target tissue after its administration is more suitable to be used as the enhancement agent for HIFU treatment according to the present invention.

The enhancement agent for HIFU treatment according to the claims greatly decreases the EEF of the target tissue after its administration. As a result, the ratio between the EEF of the target tissue measured before the administration of the enhancement agent (i.e. $EEF_{(base)}$) and the EEF of the target tissue measured after the administration of the enhancement agent (i.e. $EEF_{(measurement)}$) is more than 1, preferably more than 2, and more preferably over 4. The upper limit of the ratio is not particularly limited and a higher ratio is preferred.

Specifically, the enhancement agent for HIFU treatment of the present invention comprises a discontinuous phase comprised of a core encapsulated by a membrane-forming material and a continuous phase comprised of aqueous medium. The discontinuous phase is uniformly dispersed in the continuous phase and the particle size of the discontinuous phase ranges from 0.1-8μm, preferably 0.5-5μm and more preferably 2.5-5μm; the amount of the membrane-forming material in the enhancement agent is 0.1-100g/L, preferably 5-50g/L and more preferably 5-20g/L; the core is comprised of a liquid that does not undergo a liquid-gas phase transition at 38-100°C and is selected from a group consisting of saturated fatty acid, unsaturated fatty acid and iodized oil, and the amount of the core material in the enhancement agent is 5-200g/L, preferably 10-100g/L, and more preferably 20-80g/L.

In the above embodiment of the present invention, the membrane-forming material includes: lipids, such as 3-sn-phosphatidylcholine, 1,2-dipalmitoyl-sn-glycero-3-phosphatidylglycerol sodium salt, 1,2-distearoyl-sn-glycero-3-phosphatidylcholine, sodium 1,2-dipalmitoyl-sn-glycero-3-phosphatidate, 1,2-dipalmitoyl-sn-glycero-3-phosphatidylcholine, phosphatidylserine and hydrogenated phosphatidylserine, cholesterol, and glycolipide; saccharides, including, for example, glucose, fructose, sucrose, starch and the degradation products thereof; proteins, such as albumin, globulin, fibrinogen, fibrin, hemoglobin, and the degradation products of plant proteins and the like.

The membrane-forming material of the particle enhancement agent for HIFU treatment according to the present invention is preferably a biocompatible and degradable biomaterial, such as a lipid, such that the enhancement agent can be injected intravenously, transported through the blood circulation smoothly, and then phagocytosed quickly by the tissues of the human body, which are full of reticuloendothelial cells. Therefore, a mass of enhancement agent can be deposited in the tissues of the human body in a certain time, significantly changing the acoustic environment of the target tissue. Thus, the ultrasound absorption capacity of the tissue can be significantly enhanced, the acoustic energy deposition at the target tissue during HIFU treatment can be increased, and eventually the ability of clinical HIFU treatment to ablate tumor cells can be improved greatly.

In the above embodiment of the present invention, the aqueous medium is distilled water, physiological saline or glucose solution. The concentration of the glucose solution can be up to 50% (w/v). However, the glucose solution cannot be used as the aqueous medium for the particle enhancement agent for HIFU treatment in diabetic patients.

When oil is used as the core material, the enhancement agent may contain an emulsifier. The emulsifier is typically selected from a group consisting of ethylene glycol mono-$C_{16-18}$-fatty acid esters, diethylene glycol mono-$C_{16-18}$-fatty acid esters, diethylene glycol di-$C_{16-18}$-fatty acid esters, triethylene glycol mono-$C_{16-18}$-fatty acid esters, sorbitan fatty acid ester (Span type) emulsifiers, polysorbate (Tween type) emulsifiers, polyethylene glycol monolaurate-based emulsifiers, polyoxyethylene laurate-based emulsifiers, 3-sn-phosphatidylcholine (lecithin), cholic acid. The amount of emulsifier in the enhancement agent is 5-150g/L. In addition, the enhancement agent may also contain a stabilizing agent, such as carboxymethylcellulose sodium (CMC-Na), carboxymethylcellulose potassium, carboxyethylcellulose sodium, carboxyethylcellulose potassium, carboxypropylcellulose sodium, carboxypropylcellulose potassium, glycerin. The amount of the CMC-Na contained in the enhancement agent is 0.01-10g/L, preferably 0.05-0.6g/L, and more preferably 0.1-0.3g/L. The amount of the glycerin contained in the enhancement agent is 5-100g/L.

In a more preferred embodiment, in order to increase the stability of the enhancement agent, the enhancement agent is adjusted to pH 7.0-9.0, preferably 7.5-8.5. Inorganic or organic acids or bases may be used to adjust the pH value of the enhancement agent.

Additionally, in order to make the particle enhancement agent for HIFU treatment according to the present invention target a specific tumor tissue or focus, substances having specific affinity to the tumor tissue or the focus, such as a tumor-specific antibody, may be added to the enhancement agent.

In another embodiment, the present invention provides a method for preparing the particle enhancement agent for HIFU treatment. The method comprises:

(1) weighing and mixing a membrane-forming material and a core material to obtain 0.1-100g/L membrane forming material and 5-200g/L core material, to form an oil phase;
(2) adding an aqueous medium to the oil phase prepared in step (1) up to a predetermined volume, and stirring the mixture to form a coarse emulsion;

(3) emulsifying the coarse emulsion prepared in step (2) by sonication with a power of 300W to 500W for 30 seconds to 3 minutes.

In the method for preparation of the particle enhancement agent for HIFU treatment of the present invention, it is preferable that the membrane-forming material and the core material are fully dissolved by heating to form the oil phase in the step (1). And it is more preferable that a stabilizing agent may be added to the mixture before the membrane-forming material and the core material are fully dissolved. The aqueous medium in step (2) may contain an emulsifier.

The present invention is further directed to a method for increasing energy deposition at the target location during the HIFU treatment, wherein, the method comprises administering an effective dosage of the particle enhancement agent of the present invention intravenously via continuous and rapid IV instillation or bolus injection to a patient at 0-24h before applying HIFU treatment to a patient. The effective dosage mentioned above varies with the type of tumor, weight of patient, location of tumor, volume of tumor and the like. However, a doctor or a pharmacist can easily determine the suitable dosage for different patients. For example, the dosage can be selected from the range of 0.01-5ml/kg, preferably 0.01-2.5ml/kg.

## DETAILED DESCRIPTION OF THE INVENTION

Example 1

The following materials were mixed: 4g iodized oil for injection (purchased from Shanghai Chemical Reagent Company), 0.6g yolk lecithin for injection (purchased from Shanghai Chemical Reagent Company) and 1.25g glycerin for injection (purchased from Shanghai Chemical Reagent Company), and this mixture was dissolved and formed an oil phase after heating to 70°C. Distilled water containing 1% (w/v) F-68 emulsifier (purchased from Sigma Company) was added to the oil phase to a final volume of 17.5ml. The mixture was agitated to obtain a coarse emulsion. The coarse emulsion, which was poured into a boiling tube, was emulsified by sonication with a power of 350W for 2 minutes. The resulting uniformly emulsified iodized oil was sterilized through flowing steam at 100°C for 30 minutes. The final product had a pH of 7.5-8.5, iodine content of 0.13g/ml, particle size of less than $1\mu m$, and osmotic pressure of 350mosm/kg $H_2O$.

Examples 2 to 4

Examples 2 to 4 were prepared according to the same method and procedures described in Example 1 except that the iodized oil for injection was replaced with soybean oil for injection as the core material, and the yolk lecithin for injection was replaced with lecithin as the membrane-forming material. The particle enhancement agent for HIFU treatment of the present invention was obtained according to the formulation set forth below in Table 1. The enhancement agents were obtained as white emulsion liquids, which can be administered to animals and human beings via intravenous injection. The parameters of the products were also shown in Table 1.

Table 1

|  | Example 2 | Example 3 | Example 4 |
| --- | --- | --- | --- |
| Concentration of Soybean oil for injection in the enhancement agent (w/v) | 10% | 20% | 10% |
| Amount of Soybean oil for injection | 100g | 200g | 100g |
| Amount of Lecithin for injection | 12g | 12g | 12g |
| Amount of Glycerin for injection | 22g | 22g | 16.7g |
| Final volume after Water for injection added | 1000ml | 1000ml | 1000ml |
| pH (c.a.) | 8 | 8 | 8 |
| Particle size of the discontinuous phase | 0.1-2$\mu$m | 1-5$\mu$m | 0.5-2$\mu$m |
| Osmotic pressure (mosm/kg $H_2O$) | 300 | 350 | 310 |
| Energy MJ (kcal) | 4.6 (1100) | 8.4 (2000) | 12.6 (3000) |

The animal tests are presented below to show the effects of the enhancement agent for HIFU treatment of the present invention in combination with use of HIFU Tumor Therapeutic Devices.

Animal test 1 Combined use of the particle enhancement agent for HIFU treatment as prepared in Example 3 and HIFU Therapeutic System Model-JC

Fifty New Zealand white rabbits (about 3 months old) with no limitation on sex, which were provided by the Laboratory Animals Center of Chongqing University of Medical Sciences, were equally divided into Group A and Group B. The rabbits in Group A and Group B weighed 2.22 $\pm$ 0.21kg and 2.24 $\pm$ 0.19kg (P > 0.05), respectively.

The New Zealand white rabbits were anaesthetized through intramuscular injection, fastened to the treatment bed of a High-intensity Focused Ultrasound Tumor Therapeutic System Model-JC manufactured by Chongqing Haifu (HIFU) Technology Co. Ltd., and then treated with this System. The High-intensity Focused Ultrasound Tumor Therapeutic System Model-JC is composed of an adjustable power generator, a B-mode ultrasound monitoring system, a therapeutic transducer, a mechanical motion control system, a treatment bed, and an acoustic coupling device. The therapeutic transducer of the System, with a working frequency of 1MHz, diameter of 150mm, and focal distance of 150mm, using standard circulating degassed water with a gas content of no more than 3ppm, can produce a focal region of 2.3x2.4x26mm and deliver an average acoustic intensity of 5500W/cm$^2$.

The rabbit livers were pre-scanned by the B-mode scanner of the HIFU therapeutic system. Two slices with an interval of at least 2cm at exposure depth of 2.0cm were measured. For each rabbit of Group A, the left side of the rabbit liver (the left/middle lobe) was considered as the control lobe (which was administered with physiological saline solution), and the right side of the rabbit liver (the right lobe) was considered as the experimental lobe (which was administered with the enhancement agent for HIFU treatment as prepared in Example 3, and also called the enhancement agent side). The control lobe and experimental lobe were reversely positioned in Group B. The exposure depth of HIFU treatment (i.e., the distance from the skin surface to the focal point) was also 2.0cm. After the liver slices were chosen, the physiological saline solution was delivered via rabbit ear border vein at 50-60 drops/min. After 20 minutes, the left side of the rabbit liver (Group A) or the right side of the rabbit liver (Group B) was exposed to HIFU under single pulse exposure or multi-pulse exposure (line length: 1 cm, scanning speed: 3mm/s), and gray scale changes and exposure time in the target location were recorded. Then the focal point of the HIFU therapeutic system was moved over to the opposite site. Instead of the physiological saline solution, the enhancement agent for HIFU treatment as prepared in Example 3 was administered intravenously, the injection speed and the time being the same as that of the control lobe of liver. Then the right side of the rabbit liver (Group A) or the left side of the rabbit liver (Group B) was exposed to HIFU. The treatment modes used for both sides of the liver of the same rabbit were the same.

The rabbits were sacrificed and dissected at 24 hours after HIFU treatment. The dimensions (length, width and thickness) of the coagulation necrosis zone of the rabbit liver lesions were measured. The volume of coagulation necrosis was calculated according to the formula of V=4/3$\pi\times$1/2 length $\times$ 1/2 width $\times$ 1/2 thickness. The EEF was calculated according to the expression $EEF = \dfrac{\eta Pt}{V}$ (J/mm$^3$). The EEFs were compared between and within Group A and Group B. A substance that greatly decreases the EEF of the target tissue after its administration is more suitable to be used as the enhancement agent for HIFU treatment according to the present invention. The results are shown in Table 2.

Table 2 EEF of the control lobe and the experimental lobe

|  | Group A | Group B | Totaling | P value |
|---|---|---|---|---|
| Control lobe | 7.09 $\pm$ 4.11 | 6.67 $\pm$ 3.13 | 6.87 $\pm$ 3.60 | >0.5* |
| Experimental lobe | 2.73 $\pm$ 1.64 | 3.43 $\pm$ 2.07 | 3.10 $\pm$ 1.89 | >0.5* |
| P value | <0.001 | <0.001 | <0.00 |  |

The results in Table 2 indicate that there were no notable differences between the rabbits in Group A and those in Group B that were administered with physiological saline solution; and also that there were no notable differences between rabbits in Group A and those in Group B that were administered with the particle enhancement agent for HIFU treatment as prepared in Example 3. However, when comparing the experimental results of the control lobe with those of the experimental lobe, there were significant differences between the control lobes and the experimental lobes in Group A or in Group B. When combining the results of Group A and Group B, it could be seen that the EEF of the experimental lobe greatly decreased. In fact, the EEF of the control lobe which was administered with physiological saline solution is about 2.22 times as much as the EEF of the experimental lobe.

Animal test 2 Combined use of the emulsified iodized oil as prepared in Example 1 and HIFU Therapeutic System Model-JC

Thirty New Zealand white rabbits each weighing approximately 2kg, which were provided by the Laboratory Animals Center of Chongqing University of Medical Sciences, were divided into an experimental group and a control group randomly with 15 rabbits for each group. Two exposure spots were introduced for each rabbit. The rabbits in the control group were administered with physiological saline solution (dosage: 2.5ml/kg) by rapid injection via rabbit ear border vein. The rabbits in the experimental group were administered with emulsified iodized oil as prepared in Example 1 (dosage: 2.5ml/kg) by rapid injection via rabbit ear border vein followed by flushing with 1ml physiological saline solution in order to ensure that the emulsified iodized oil had entered into the body completely. One hour later, a High-intensity Focused Ultrasound Tumor Therapeutic System Model-JC manufactured by Chongqing Haifu (HIFU) Technology Co. Ltd. was used to radiate the livers of the white rabbits in the experimental group and the control group under single pulse exposure. The power for exposure was 220W; the frequency was 1.0MHZ; the exposure depth was 20mm and the exposure was stopped when coagulative necrosis occurred. The measured data were expressed with mean value $\pm$ SD, processed by the statistics software SPSS 10.0 for Windows using independent and paired sample tests. The enumeration data was determined by using chi-square ($\chi^2$) test. The comparisons between the EEF of the control group and the EEF of the experimental group were shown in Table 3.

Table 3 Comparisons between the EEFs of the control group and the experimental group

| Group | N | EEF ($\chi \pm s$) (J/mm$^3$) |
|---|---|---|
| Control group | 30 | $31.05 \pm 2.68$ |
| Experimental group | 30 | $7.16 \pm 1.38$* |

N refers to the numbers of the exposure spots.
* $P < 0.001$ when compared to the control group.

The results in Table 3 show that the emulsified iodized oil as prepared in Example 1 could greatly reduce the level of EEF for causing lesions of the hepatic tissue with HIFU treatment.

Animal test 3 *In vitro* study of the enhancement agent as prepared in Example 3

Ten New Zealand white rabbits (about 3 months old) with no limitation on sex, which were provided by the Laboratory Animals Center of Chongqing University of Medical Sciences, were randomly divided into an experimental group (which was administered with enhancement agent for HIFU treatment as prepared in Example 3) and a control group (which was administered with physiological saline solution). The rabbits in the two groups weighed $2.40 \pm 0.45$kg and $2.32 \pm 0.08$kg ($P > 0.5$), respectively. These rabbits were fasted for 24 hours before experiments. HIFU gynaecological therapeutic apparatus CZF-1 manufactured by Chongqing Haifu (HIFU) Technology Co. Ltd. was used to radiate the rabbit livers. The HIFU gynaecological therapeutic apparatus CZF-1 was composed of a power source, an applicator, and circulating water as described in Chinese Patent No. 01144259.X. The parameters in this test were set up as follows: power: 4.05W; frequency: 11MHz; and pulse: 1000Hz.

After the white rabbits were anaesthetized through intramuscular injection, the enhancement agent for HIFU treatment as prepared in Example 3 was delivered via rabbit ear border vein to the rabbits in the experimental group at 50-60 drops/minute for 20 minutes and the physiological saline solution was delivered to those in the control group at 50-60 drops/minute for 20 minutes.

One hour after transfusion, the rabbit was fastened to a workbench in supine position. For each rabbit, the laparotomy was carried out with a 4-5cm incision in the midsection and the rabbit liver in the abdominal cavity was exposed and pulled out slightly after the abdomen wall was opened layer by layer. One or two exposure spots on each liver lobe were introduced for each exposure duration of 3s, 6s, and 9s. The experiments were carried out using the parameters as mentioned above after the exposure spots were introduced. After the lesions were generated, the rabbit liver was put back to the abdominal cavity and the abdomen wall was sutured layer by layer.

The next day, the rabbits were sacrificed with excessive anaesthetization. The livers were removed and photographed. The dimensions of lesions were measured and the EEF was calculated. All data was expressed with mean value $\pm$ SD, processed by the statistics software SPSS 10.0 for Windows and the independent sample test. The statistics was significant when P value was less than 0.05. In this test, 21 exposure spots for each exposure duration of 3s, 6s, and 9s and 63 ($21 \times 3$) exposure spots in total were obtained in the control group; 30 exposure spots for each exposure duration of 3s, 6s, and 9s and 90 ($30 \times 3$) exposure spots in total were obtained in the experimental group. The EEF was calculated with the above-mentioned expression, and the results are shown in Table 4.

Table 4 The EEF of the control group and the experimental group

| | | Exposure duration | | |
|---|---|---|---|---|
| | n | 3s | 6s | 9s |
| Control group | 21 | 0.2749± 0.2409 | 0.1783± 0.0733 | 0.1846± 0.0896 |
| Experimental group | 30 | 0.1177± 0.0609 | 0.1367± 0.0613 | 0.1463± 0.069 |
| P Value | | < 0.01 | < 0.05 | > 0.05 |

The results in Table 4 show that the EEF for each exposure duration of 3s, 6s, and 9s in the control group were 2.34, 1.30 and 1.26 times of the EEF for each exposure duration of 3s, 6s, and 9s in the experimental group, respectively. In fact, the mean of the EEF in the control groups (3s, 6s, and 9s) is 1.59 times as much as the mean of the EEF in the experimental groups (3s, 6s, and 9s). If the data obtained in the exposure duration of 9s, which the difference of the EEF between the control group and the experimental group was not statistically significant, were not considered, the mean of the EEF in the control groups (3s, and 6s) is 1.78 times as much as the mean of the EEF in the experimental groups (3s, and 6s).

**INDUSTRIAL APPLICABILITY**

The particle enhancement agent for HIFU treatment of the present invention can change the acoustic environment of the target location greatly and can reduce the acoustic energy needed to cause lesions of a target tissue (tumor/non-tumor tissue) per unit volume of the tissue during HIFU treatment. Accordingly, deep-seated and large-sized tumors can be treated with HIFU treatment more effectively under a certain acoustic power without damaging the normal tissues along the acoustic pathway. It becomes possible to use the enhancement agent for HIFU treatment of the present invention to treat a patient with hepatic tumor that is blocked by the ribs without removal of the ribs.

**Claims**

1. An enhancement agent for use in high intensity focused ultrasound (HIFU) treatment, wherein, the enhancement agent comprises a discontinuous phase composed of a core material encapsulated by a membrane-forming material, and a continuous phase comprised of aqueous medium, the discontinuous phase is uniformly dispersed in the continuous phase and the particle size of the discontinuous phase ranges from 0.1-8$\mu$m; the amount of the membrane-forming material in the enhancement agent is 0.1-100g/L; the core material is comprised of a liquid that does not undergo a liquid gas phase transition at 38-100°C and is selected from a group consisting of saturated fatty acid, unsaturated fatty acid and iodized oil, and the amount of the core material in the enhancement agent is 5-200g/L.

2. The enhancement agent according to claim 1, wherein the discontinuous phase has particle size ranging from 0.5-5$\mu$m.

3. The enhancement agent according to claim 2, wherein the discontinuous phase has particle size ranging from 2.5-5$\mu$m.

4. The enhancement agent according to claim 1, wherein the membrane-forming material is one or more substances selected from a group consisting of lipids, cholesterol and glycolipide.

5. The enhancement agent according to claim 4, wherein the membrane-forming material comprises phospholipid selected from a group consisting of 3-sn-phosphatidylcholine, 1,2-dipalmitoyl-sn-glycero-3-phosphatidylglycerol sodium salt, 1,2-distearoyl-sn-glycero-3-phosphatidylcholine, sodium 1,2-dipalmitoyl-sn-glycero-3-phosphatidate, 1,2-dipalmitoyl-sn-glycero-3-phosphatidylcholine, phosphatidylserine and hydrogenated phosphatidylserine.

6. The enhancement agent according to claim 1, wherein the amount of the membrane-forming material in the enhancement agent is 5-50g/L.

7.  The enhancement agent according to claim 6, wherein the amount of the membrane-forming material in the enhancement agent is 5-20g/L.

8.  The enhancement agent according to claim 1, wherein the core material comprises soybean oil.

9.  The enhancement agent according to claim 1, wherein the core material comprises iodized oil.

10. The enhancement agent according to claim 1, wherein the enhancement agent contains an emulsifier in an amount of 5-150g/L, the emulsifier is selected from a group consisting of ethylene glycol mono-$C_{16-18}$-fatty acid esters, diethylene glycol mono-$C_{16-18}$-fatty acid esters, diethylene glycol di-$C_{16-18}$-fatty acid esters, triethylene glycol mono-$C_{16-18}$-fatty acid esters, sorbitan fatty acid esters, polysorbate, polyethylene glycol monolaurate, polyoxyethylene laurate, 3-sn-phosphatidylcholine, and cholic acid.

11. The enhancement agent according to claim 1, wherein the aqueous medium comprises distilled water, physiological saline solution or glucose solution.

12. The enhancement agent according to claim 1, wherein the amount of the core material in the enhancement agent is 10-100g/L.

13. The enhancement agent according to claim 12, wherein the amount of the core material in the enhancement agent is 20-80g/L.

14. The enhancement agent according to any one of claims 1-13, wherein the enhancement agent contains a stabilizing agent comprising carboxymethylcellulose sodium; the amount of the carboxymethylcellulose sodium in the enhancement agent is 0.01-10g/L.

15. The enhancement agent according to any one of claims 1-13, wherein the enhancement agent contains a stabilizing agent comprising glycerin; the amount of the glycerin in the enhancement agent is 5-100g/L.

16. Use of a lipid emulsion, which is used for preparing the enhancement agent for HIFU treatment.

17. The use of the lipid emulsion according to claim 16, wherein the lipid emulsion is fat emulsion.

18. The use of the lipid emulsion according to claim 16, wherein the lipid emulsion is emulsified iodized oil.

**Patentansprüche**

1.  Wirkverstärkermittel zur Verwendung bei einer Behandlung mit fokussiertem Ultraschall hoher Intensität (HIFU; High Intensity Focused Ultrasound), wobei das Wirkverstärkermittel eine diskontinuierliche Phase umfasst, die ein Kernmaterial eingekapselt in einem membranbildenden Material umfasst, sowie eine kontinuierliche Phase, die ein wässriges Medium umfasst, wobei die diskontinuierliche Phase gleichmäßig in der kontinuierliche Phase verteilt ist und die Partikelgröße der diskontinuierlichen Phase im Bereich von 0,1 bis 8 $\mu$m liegt, wobei die Menge an membranbildendem Material in dem Wirkverstärkermittel 0,1 bis 100 g/l beträgt, wobei das Kernmaterial eine Flüssigkeit umfasst, bei der zwischen 38 und 100 °C kein Phasenübergang von flüssig zu gasförmig erfolgt und die aus einer Gruppe ausgewählt ist, die gesättigte Fettsäure, ungesättigte Fettsäure und iodiertes Öl umfasst, und wobei die Menge an Kernaterial in dem Wirkverstärkermittel 5 bis 200 g/l beträgt.

2.  Wirkverstärkermittel nach Anspruch 1, wobei die diskontinuierliche Phase eine Partikelgröße im Bereich von 0,5 bis 5 $\mu$m aufweist.

3.  Wirkverstärkermittel nach Anspruch 2, wobei die diskontinuierliche Phase eine Partikelgröße im Bereich von 2,5 bis 5 $\mu$m aufweist.

4.  Wirkverstärkermittel nach Anspruch 1, wobei das membranbildende Material eine oder mehrere Substanzen umfasst, die aus einer Gruppe ausgewählt ist/sind, die Lipiden, Cholesterol und Glycolipid umfasst.

5.  Wirkverstärkermittel nach Anspruch 4, wobei das membranbildende Material Phospholipid umfasst, das aus einer Gruppe ausgewählt ist, umfassend aus 3-sn-Phosphatidylcholin, 1,2-Dipalmitoyl-sn-glycero-3-phosphatidylglycerol-Natriumsalz, 1,2-Distearoyl-sn-glycero-3-phosphatidylcholin, Natrium-1,2-dipalmitoyl-sn-glycero-3-phosphatidat, 1,2-Dipalmitoyl-sn-glycero-3-phosphatidylcholin, Phosphatidylserin und hydriertes Phosphatidylserin.

6. Wirkverstärkermittel nach Anspruch 1, wobei die Menge an membranbildendem Material in dem Wirkverstärkermittel 5 bis 50 g/l beträgt.

7. Wirkverstärkermittel nach Anspruch 6, wobei die Menge an membranbildendem Material in dem Wirkverstärkermittel 5 bis 20 g/l beträgt.

8. Wirkverstärkermittel nach Anspruch 1, wobei das Kernmaterial Sojabohnenöl umfasst.

9. Wirkverstärkermittel nach Anspruch 1, wobei das Kernmaterial iodiertes Öl umfasst.

10. Wirkverstärkermittel nach Anspruch 1, wobei das Wirkverstärkermittel einen Emulgator in einer Menge von 5 bis 150 g/l umfasst, wobei der Emulgator aus einer Gruppe ausgewählt ist, umfassend Ethylenglycol-mono-$C_{16-18}$-Fettsäureester, Diethylenglycol-mono-$C_{16-18}$-Fettsäureester, Diethylenglycol-di-$C_{16-18}$-Fettsäureester, Triethylenglycol-mono-$C_{16-18}$-Fettsäureester, SorbitanFettsäureester, Polysorbat, Polyethylenglycolmonolaurat, Polyoxyethylenlaurat, 3-sn-Phosphatidyl-cholin und Cholinsäure.

11. Wirkverstärkermittel nach Anspruch 1, wobei das wässrige Medium destilliertes Wasser, physiologische Kochsalzlösung oder Glucoselösung umfasst.

12. Wirkverstärkermittel nach Anspruch 1, wobei die Menge an Kernmaterial in dem Wirkverstärkermittel 10 bis 100 g/l beträgt.

13. Wirkverstärkermittel nach Anspruch 12, wobei die Menge an Kernmaterial in dem Wirkverstärkermittel 20 bis 80 g/l beträgt.

14. Wirkverstärkermittel nach einem der Ansprüche 1 bis 13, wobei das Wirkverstärkermittel ein Stabilisierungsmittel enthält, das Carboxymethylcellulose-Natrium umfasst, wobei die Menge an Carboxymethylcellulose-Natrium in dem Wirkverstärkermittel 0,01 bis 10 g/l beträgt.

15. Wirkverstärkermittel nach einem der Ansprüche 1 bis 13, wobei das Wirkverstärkermittel ein Stabilisierungsmittel enthält, das Glycerin umfasst, wobei die Menge an Glycerin in dem Wirkverstärkermittel 5 bis 100 g/l beträgt.

16. Verwendung einer Lipidemulsion, die zur Darstellung des Wirkverstärkermittels für eine HIFU-Behandlung genutzt wird.

17. Verwendung der Lipidemulsion nach Anspruch 16, wobei die Lipidemulsion eine Fettemulsion ist.

18. Verwendung der Lipidemulsion nach Anspruch 16, wobei die Lipidemulsion ein emulgiertes, iodiertes Öl ist.

**Revendications**

1. Agent d'amplification destiné à être utilisé dans un traitement ultrasonique focalisé à haute intensité (HIFU), l'agent d'amplification comprenant une phase discontinue constituée d'une matière centrale encapsulée par une matière de formation de membrane, et une phase continue constituée d'un milieu aqueux, la phase discontinue étant dispersée de manière uniforme dans la phase continue et le diamètre de particule de la phase discontinue allant de 0,1 à 8 $\mu$m ; la quantité de la matière de formation de membrane dans l'agent d'amplification étant de 0,1 à 100 g/l ; la matière centrale étant constituée d'un liquide qui ne subit pas de transition de phase liquide-gaz à 38-100°C et étant choisie dans un groupe consistant en un acide gras saturé, un acide gras insaturé et une huile iodée, et la quantité de la matière centrale dans l'agent d'amplification étant de 5 à 200 g/l.

2. Agent d'amplification suivant la revendication 1, dans lequel la phase discontinue a un diamètre de particule allant de 0,5 à 5 $\mu$m.

3. Agent d'amplification suivant la revendication 2, dans lequel la phase discontinue a un diamètre de particule allant de 2,5 à 5 μm.

4. Agent d'amplification suivant la revendication 1, dans lequel la matière de formation de membrane consiste en une ou plusieurs substances choisies dans un groupe consistant en des lipides, le cholestérol et un glycolipide.

5. Agent d'amplification suivant la revendication 4, dans lequel la matière de formation de membrane comprend un phospholipide choisi dans un groupe consistant en la 3-sn-phosphatidylcholine, le sel de sodium de 1,2-dipalmitoyl-sn-glycéro-3-phosphatidylglycérol, la 1,2-distéaroyl-sn-glycéro-3-phosphatidylcholine, le 1,2-dipalmitoyl-sn-glycéro-3-phosphatidate de sodium, la 1,2-dipalmitoyl-sn-glycéro-3-phosphatidylcholine, la phosphatidylsérine et la phosphatidylsérine hydrogénée.

6. Agent d'amplification suivant la revendication 1, dans lequel la quantité de la matière de formation de membrane dans l'agent d'amplification est de 5 à 50 g/l.

7. Agent d'amplification suivant la revendication 6, dans lequel la quantité de la matière de formation de membrane dans l'agent d'amplification est de 5 à 20 g/l.

8. Agent d'amplification suivant la revendication 1, dans lequel la matière centrale comprend de l'huile de soja.

9. Agent d'amplification suivant la revendication 1, dans lequel la matière centrale comprend une huile iodée.

10. Agent d'amplification suivant la revendication 1, l'agent d'amplification contenant un émulsionnant en une quantité de 5 à 150 g/l, l'émulsionnant étant choisi dans un groupe consistant en des mono-esters d'acides gras en $C_{16}$ à $C_{18}$ d'éthylèneglycol, des mono-esters d'acides gras en $C_{16}$, à $C_{18}$ de diéthylèneglycol, des diesters d'acides gras en $C_{16}$ à $C_{18}$ de diéthylèneglycol, des mono-esters d'acides gras en $C_{16}$ à $C_{18}$ de triéthylèneglycol, des esters d'acides gras de sorbitanne, un polysorbate, le monolaurate de polyéthylèneglycol, le laurate de polyoxyéthylène, la 3-sn-phosphatidylcholine et l'acide cholique.

11. Agent d'amplification suivant la revendication 1, dans lequel le milieu aqueux comprend l'eau distillée, une solution saline physiologique ou une solution de glucose.

12. Agent d'amplification suivant la revendication 1, dans lequel la quantité de matière centrale dans l'agent d'amplification est de 10 à 100 g/l.

13. Agent d'amplification suivant la revendication 12, dans lequel la quantité de matière centrale dans l'agent d'amplification est de 20 à 80 g/l.

14. Agent d'amplification suivant l'une quelconque des revendications 1 à 13, ledit agent d'amplification contenant un stabilisant comprenant de la carboxyméthylcellulose sodique ; la quantité de carboxyméthylcellulose sodique dans l'agent d'amplification étant de 0,01 à 10 g/l.

15. Agent d'amplification suivant l'une quelconque des revendications 1 à 13, l'agent d'amplification contenant un stabilisant comprenant du glycérol ; la quantité de glycérol dans l'agent d'amplification étant de 5 à 100 g/l.

16. Utilisation d'une émulsion lipidique, qui est utilisée pour la préparation de l'agent d'amplification pour le traitement HIFU.

17. Utilisation de l'émulsion lipidique suivant la revendication 16, dans laquelle l'émulsion lipidique est une émulsion de matière grasse.

18. Utilisation de l'émulsion lipidique suivant la revendication 16, dans laquelle l'émulsion lipidique est une huile iodée émulsionnée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 01144259X

**Non-patent literature cited in the description**

- Journal of Cancer Research and Clinical Oncology. Springer-Verlag, 1997, vol. 123, 639-644

- **Baoqin Liu et al.** *Chinese Journal of Ultrasound in Medicine,* 2002, vol. 18 (8), 565-568